# EUROPEAN PATENT APPLICATION

(11) **EP 2 404 579 A1**
(43) Date of publication of application: **11.01.2012**
(21) Application number: 10748368.7
(22) Date of filing: 26.02.2010
(51) Int. Cl.: A61F 5/453, A61B 10/00, A61J 1/05, B65D 41/50, B65D 51/20, G01N 1/10

(54) **DEVICE FOR COLLECTING SEMEN SAMPLES**

(30) Priority: 02.03.2009 ES 200900407 U
(71) Applicant: Sánchez Serrano, María Cruz, 46015 Valencia (ES); Depraetere, Johan Patrick Achiel Marie, 46015 Valencia (ES); Vazquez Mora, Marcelino, 46015 Valencia (ES); Escobedo Lucea, María Del Carmen, 46026 Valencia (ES); Ayuso Sacido, Ángel, 46011 Valencia (ES)
(72) Inventor: Sánchez Serrano, María Cruz, 46015 Valencia (ES); Depraetere, Johan Patrick Achiel Marie, 46015 Valencia (ES); Vazquez Mora, Marcelino, 46015 Valencia (ES); Escobedo Lucea, María Del Carmen, 46026 Valencia (ES); Ayuso Sacido, Ángel, 46011 Valencia (ES)
(74) Representative: Ungria Lopez, Javier
(86) International application number: PCT/ES2010/070107
(87) International publication number: WO 2010/100306

(57) **Abstract**

The invention relates to a device for collecting semen samples, which is configured such that the patient/donor can perform ejaculation and the corresponding semen collection in his own home, for subsequent transfer to a medical centre or laboratory where the semen can be analyzed. The invention is **characterized in that** it consists of a preferably plastic cylindrical tube including mouth that is provided with a peripheral flange having a rounded edge in order to allow the condom into which the patient/donor ejaculates to be securely fastened thereto, and a closure cover that is screwed onto the cylindrical tube in order to press and secure the semen-filled condom housed inside said tube.

## Description

### OBJECT OF THE INVENTION

The present invention, as expressed in the title of this specification, refers to a device for collecting semen samples, which has been designed and constructed in order to facilitate the semen collection process for the patient/donor, from any method to reach orgasm, also enabling its collection through sexual intercourse, and achieving that the sample arrives in the best conditions to the laboratory or medical center where the corresponding analysis will be performed.

The object of the invention is to facilitate said process for collecting the semen without affecting the viability/quality/sperm count, further achieving to minimize the risk of contagion for professionals in reproductive medicine during the reception and preliminary sample preparation, as well as preventing the loss of sample and facilitating the secure transport. In this sense, the device will also allow facilitating the collection of samples from patients suffering from a disease, treatment of which will harm their gonadal function (i.e. his subsequent sperm production) and for that reason have decided to preserve (freeze-cryopreserved) his semen.

### BACKGROUND OF THE INVENTION

As is known, the collection and transfer of semen samples must meet certain conditions, both by the patient/donor and by the collection container in order to achieve optimal final results after the corresponding analysis.

In this sense, the semen sample is obtained by masturbation and ejaculation into a glass or plastic container, so that in case of being made of plastic a test should be performed for determining the toxicity degree thereof, the container itself must be tempered in order to avoid risk of hypothermia. In case of requiring a microbiological analysis, the patient/donor should urinate and wash hands and genitals prior to obtaining the ejaculate.

Moreover, the ejaculation for obtaining the sample is usually performed in a place close to the laboratory or medical center, which involves nervousness for the patient/donor and logical stress, whereby the conditions are not the most favorable for the patient/donor to feel comfortable and can perform the ejaculation with full guarantees.

### DESCRIPTION OF THE INVENTION

The device of the invention is intended to solve the drawbacks and problems shown by current systems for collecting and processing semen samples, being based on that the device consists of a preferably plastic tube, without excluding other appropriate materials, which tube has in its mouth a peripheral flange with a fully rounded outer edge, and in a section slightly apart from said flange a thread for screwing a closure cover which may be provided on its inner surface with a sterile sheet.

The patient/donor will ejaculate at his home into a condom made of a material not harmful to the sperm which will carefully be inserted inside the tube, being positioned by gravity because of the weight of the semen, and retained on the outer edge of the peripheral wing of the tube when screwed until locking the cover itself.

In this way the patient/donor or any other person may carry semen to the medical center or laboratory for completing the relevant analysis.

The action for extracting the container and treating the sample by medical personnel or specialized analysts, will take place in a completely sterile manner, since is not necessary to detach the cover but tearing the punched sector thereof, which has a split flange, so that once torn this independent sector may be introduced in a pipette or similar element to first go through a second thin layer, poorly resistant and waterproof on the internal face and, secondly, to extract the semen sample contained in the semen-filled condom housed inside in the tube, all without contacting with the condom or the tube interior.

The cylindrical tube may have a size adjusted to the standard size of the support racks of the so-called "centrifuges" in order that, prior to the actuation of the medical specialist on the sample contained inside said tube, this can be subjected to a slight centrifugal operation so as to stabilize the sample on the bottom of the condom, and thus facilitating to the maximum the extraction of said sample by the medical specialist.

It is also envisaged that the container can be manufactured with a double wall, preferably of polymeric material, in order to extend to the maximum the preservation period of the sample from the collection by the patient/donor to the reception and subsequent treatment by the medical specialist...

As the tube itself, it may be made of an opaque material in order to achieve a greater safeguard of the sample as well as greater discretion when carrying the device with the sample already collected by the user himself, or a transparent material providing greater visibility to the medical specialist when operating inside the tube with the pipette or the like for extracting the sample.

As to the cover, it can be sized so that it can fit the dimensions of the conventional urine sample collection containers.

Finally say that on the side surface of the cylindrical body of the device legends can be printed for indicating, for example, personal data of the patient/donor, as well as for marking the OK guidance of the cylindrical tube itself.

### BRIEF DESCRIPTION OF THE DRAWINGS

To complement the description that will be made bellow and in order to help to a better understanding of the features of the invention, a set of drawings is attached to the present specification, based on which the innovations and advantages of device for collecting semen according to the object of the invention will be more easily understood.

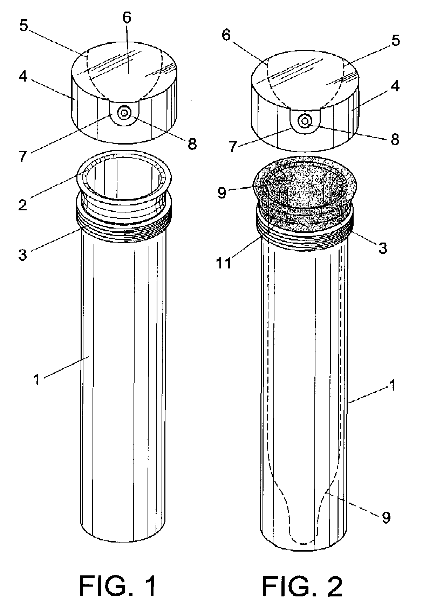

**Figure 1****.-** Shows an exploded view of the cylindrical tube of the cover in the position for being screwed thereon.
**Figure 2****.-** Shows a perspective view of the tube with the semen-filled condom inside the tube itself.

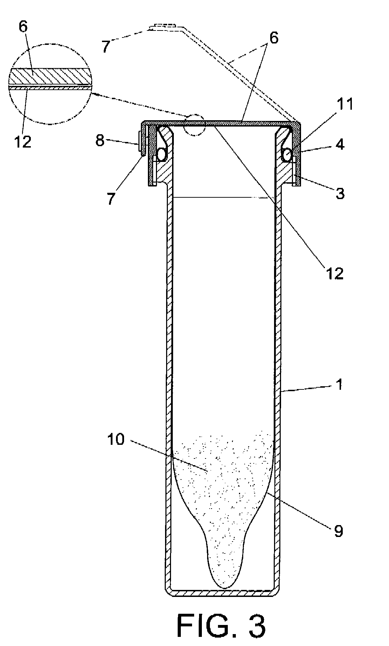

**Figure 3****.-** Shows in section the coupling of the cover onto the tube, the condom being retained over its top onto the mouth of the tube, also showing with the dashed line the opening of the tearable sector of the cover.

### DESCRIPTION OF THE PREFERRED EMBODIMENT

As can be seen in the referred figures , the device for collecting semen samples object of the invention comprises a preferably plastic cylindrical tube 1, mouth of which has a peripheral flange 2 with fully rounded edge, and below it a thread section 3. Said tube 1 is complemented with a closure cover 4 which is screwed on the section 3 of the cylindrical tube 1 itself, which cover 4 has a die-cutting 5 that defines a tearable sector 6 by pulling a tab 7, which is at rest and secures the side surface of the body of the cover 4 by means of a pressure clip 8. At the bottom of the cover 4 a sheet 8 of an easily drillable material 12 is provided, with waterproof underside.

According to these features, when the patient/donor ejaculates on a condom 9, it is inserted into the cylindrical tube 1 and by gravity, and due to own weight of the semen sample 10 ejaculated on the condom 9, it is housed inside the tube 1, leaving the top edge 11 thereof onto the peripheral and rounded flange 2 of the mouth of the cylindrical body 1, so that the condom 9 housed inside the cylindrical body 1 is secured and fixed by the closing the corresponding cover 4, when this is screwed until completing its locking, by pressing the bottom of such cover 4 against the top edge of the mouth of the flange 2 of the cylindrical body 1 and of course by pressing the mouth of the condom 9 so that it is securely fastened.

In this way, the semen sample 10 will be taken to the medical center or laboratory for analysis, making the extraction of the semen 10 previously tearing by die-cutting 5 the sector 6 of the corresponding cover 4, which tearing is performed by pulling the tab 7, so that the medical personnel insert a pipette or other similar element, going through the second layer or sheet 12, being easy to drill and insulated on the underside, so as to continue with the extraction of the corresponding semen collection 10.

## Claims

1. Device for collecting semen samples, which is configured so as the patient/donor can perform the ejaculation and semen collection in his own home for subsequent transfer to the medical center or laboratory for analyzing said semen, **characterized in that** it consists of a preferably plastic cylindrical tube (1), having a mouth equipped with a peripheral flange (2) of rounded edge for allowing an effective securing of a condom (9) in which the patient/donor makes ejaculation, also including a closure cover (4) which is screwed onto the cylindrical tube (1) for pressing and securing the semen (10) filled condom (9) housed inside the cylindrical tube (1).

2. Device for collecting semen samples, according to claim 1, **characterized in that** the cover (4) has a tearable sector (6) having a split tongue (7), in order to achieve an opening by setting apart said tearable sector (6) and allowing the introduction of a pipette for taking the semen sample (10) contained inside the condom (9), by piercing a thin layer or sheet (12) not die-cut and located on the underside of the cover (4) and attached thereto through the sides, with waterproof underside, the operation being performed without contacting with the condom (9) or the tube itself (1).

3. Device for collecting semen samples, according to previous claims, **characterized in that** the inner surface of the cover (4) preferably includes a sterile sheet.

4. Device for collecting semen samples, according to claim 1, **characterized in that**, optionally, the dimensions of the cylindrical tube (1) correspond to the standard sizes of support racks of conventional "centrifuges".

5. Device for collecting semen samples, according to claim 1, **characterized in that**, optionally, the cylindrical tube (1) is preferably double-walled made of polymeric material in order to achieve extending the preservation of the semen samples (10) therein.

6. Device for collecting semen samples, according to claims 1, 4 and 5, **characterized in that** cylindrical tube (1) is made of a transparent and/or translucent material.

7. Device for collecting semen samples, according to claims 1, 4 and 5, **characterized in that** the cylindrical tube (1) is made of an opaque material.

8. Device for collecting semen samples, according to claims 1 to 3, **characterized in that** the cover (4) is sized to allow its adaptation and attachment to conventional containers for collecting urine samples.
